# EUROPEAN PATENT APPLICATION

(11) **EP 0 725 134 A2**
(43) Date of publication of application: **07.08.1996**
(21) Application number: 96100776.2
(22) Date of filing: 19.01.1996
(51) Int. Cl.: C12M 3/06

(54) **Flexible bioreactor for therapeutic cells**

(30) Priority: 03.02.1995 US 383516
(71) Applicant: NPBI Nederlands Produktielaboratorium voor Bloedtransfusieapparatuur en Infusievloeistoffen B.V., NL-7881 HM Emmer Compascuum (NL)
(72) Inventor: van der Heiden, Johannes, 9725 LD Groningen (NL); Hilbrink, Hubertus Eduard, 7827 BL Emmen (NL)
(74) Representative: Masch, Karl Gerhard, Dr.

(57) **Abstract**

A flat flexible container forms a bioreactor for cell cultivation. The relatively small volume cell compartment is defined between a gas-permeable outer wall and a semipermeable membrane while the large volume compartment for the culture medium is defined between the semipermeable membrane and another gas-permeable outer wall. Oxygen is supplied from the exterior and outwardly diffusing carbon dioxide is removed.

## Description

### FIELD OF THE INVENTION

The present invention relates to a bioreactor for the *ex vivo* culture of somatic cells for therapeutic use.

For several therapeutic treatments cells are cultured outside the human body. Culturing can take place to increase the number of cells, to activate cells, to differentiate the cells or to change specific functions of the cells.

For the *ex vivo* culture it is essential to supply cells with adequate amounts of nutrients and dissolved oxygen and to remove waste products before they accumulate to such a level that they can harm the cells in the culture. The main nutrients are glucose (the main energy sources, which is metabolized under influence of oxygen to lactate), amino acids, electrolytes and vitamins. Generally, they are supplied in a basic medium supplemented with a buffer such as sodium bicarbonate or HEPES to keep the pH around 7.2-7.4. Also, hormones, growth factors, proteins and/or serum can be added to the medium. The main waste products are lactate, the metabolite of glucose, and CO₂. Thus CO₂ must be removed and oxygen must continuously be supplied to the cells. In many culture systems, dissolved oxygen is a limiting factor for culturing of the cells.

Refreshment of the medium can be arranged by regular changes of the medium for the removal of waste products and the supply of new nutrients. Alternatively, a steady inflow of new medium and concurrently a steady outflow of medium can be arranged. The requirements for the supply of nutrients and oxygen and for the removal of waste products depend on the metabolic activities of the cells, the kind of cells and the number of cells in the cell preparation.

Although in a small volume of medium toxic waste products can easily accumulate and nutrients are quickly exhausted, it is generally desirable to keep the cell suspension in a volume as small as possible to ease the handling during culturing and harvesting.

Cells are usually incubated at 32-37°C under gentle agitation to keep the medium and the cells homogeneous for nutrients, waste products and oxygen. Cell suspensions should not be shaken vigorously: the shear stress that is thus induced on the cells may lead to damage to the cell membrane and thus to critical cell functions.

Human cells can be cultured for several medical treatments. Examples include the culture and activation of T lymphocytes for immunotherapy for cancer, the culture of genetically changed cells for gene therapy and the expansion of blood stem cells *ex vivo*. The general procedure is that cells are collected from the patient or a donor, treated outside the body, incubated (at controlled temperature, oxygen/CO₂ environment) in a growth supporting medium and injected into a patient. It is essential that during this treatment the quality of the cell preparation in terms of number and viability of cells and prevention of contamination is kept on a level as high as possible. If a contaminating micro-organism is introduced it will usually outgrow the human cells and spoil the cell preparation. Therefore, safe procedures that can be validated must be followed with a minimum of aseptic handlings and with culture systems that are reliable and easy to use.

Several systems are known for the culture of cells.

A commonly used system exists of a tank or bottle which is partly filled with the cells and the culture medium. Oxygen is supplied in the head space of the bottle or tank. Such a system is generally not suited for the culture of cells as there is an inadequate supply of oxygen and an inhomogeneous distribution of nutrients and waste products in the vessel. Also, aseptic procedures are difficult to perform in this product concept. Therefore, such a system does not meet the quality requirements for a culture system for cells for human use. To get a better supply of oxygen and a more homogeneous distribution of waste products and nutrients, the tank can be stirred or the bottle can be rolled. However, this imposes shear stress on the cells, to which most animal cells are very sensitive.

Alternatively, flexible bags can be used for the culture of cells. These bags can be standard blood bags manufactured from f.i. PVC or they can be made of highly gas-permeable plastics, such as bags for platelet storage. An example is a bag as distributed under the name of Life-Cell Culture Flask by Baxter Healthcare Corp. Generally, these bags are simply filled with the cell suspension and the medium and put in an incubator. The medium is changed when necessary.

Bags are easy to handle and they have a relatively large surface area for gas exchange. However, cells will settle on the bottom, leading to a long diffusion path for the dissolved oxygen. Thus oxygen soon becomes the limiting factor for growth and the number of cells that can be cultured in such containers is relatively small. Many containers may therefore be needed to treat one patient resulting in many aseptic handlings. Consequently, harvesting is complicated, as extensive concentration of the cells from many bags is needed to reduce the volume of the cultured cells to a volume that can be injected into a patient. Also, such a system is expensive as large volumes of culture medium and added substances are required.

Alternatively, hollow fiber systems may be used for the culture of cells, such as marketed in the USA by Endotronics, Inc. of Minneapolis under the name of AcuSyst. These systems comprise a cartridge containing hollow fibers, defining an intra-capillary space and an extra-capillary space. In the extra-capillary space the cells are cultured. The intra- and extra-capillary space are divided by a semipermeable membrane, which usually has a cut-off at around 10 kD, preventing molecules larger than that size to pass the membrane. A constant flow of oxygenated medium is pumped through the intra-capillary space, feeding the cells with nutrients and oxygen. Waste products diffuse from the extra-capillary space to the intra-capillary space over the semipermeable membrane and are thus removed from the direct environment of the cells. In this way, a very high cell density can be reached. Large and usually expensive molecules such as hormones, growth factors and serum can be kept in the extra-capillary space, leading to considerable cost saving. A major drawback of this system is the inhomogeneity of the medium in the cartridge. Cells that are at the inlet will receive a very rich medium, while cells that are in the back (from the membrane) or at the outlet receive a poor medium with accumulated waste products. This gradient of nutrients and waste products in the cartridge leads to differential growth of the cells. Such systems are therefore very hard to validate when a homogeneous cell population is required for therapy. An additional disadvantage is that the system requires regular attention of an operator as a constant in- and outflow of medium must be maintained.

In the German patents DE 4229325 and 4229334 a system is described comprising a roller bottle having two compartments, separated by a semipermeable membrane. The upper compartment contains the basic grown medium and supplies the nutrients to the cells via the semipermeable membrane. It is fitted with a membrane for gas exchange of the medium. The medium removes the waste products. The lower, small compartment contains the cells. The smaller compartment is fitted with a silicone membrane for oxygen supply of the cells. The bottle is rotated during use, for optimum exchange of gases and nutrients and waste products. As it is a rigid system, it is very sensitive to the build-up of pressure which may happen due to heating of the medium and release of CO₂. During incubation, the pressure must be released after medium changes and the system must be handled very carefully, especially during sampling.

Another system, as marketed in the USA under the name Acumouse by Endotronics of Minneapolis, employs culturing bags with gas-permeable walls, made of silicone. A medium flows through hollow fibers in the bags, while the cells are cultured in the extra-capillary space. The bag is placed in a cassette, through which gas is pumped. In these kind of systems inhomogeneity of the culture medium and consequently of the cells may occur. As a constant flow of medium is essential, it requires regular attention by an operator.

US Patent 4661455 describes a double bag including an inner bag made of a highly gas permeable membrane incorporated in a special bag made of an ultra- or micro-filtration membrane. The cells are located between the membranes. The inner bag is oxygenated. This device is put in a separate housing in which the medium is supplied. Generally, it is very difficult to keep such systems sterile. Furthermore, they require regular attention by an operator to maintain the medium- and the gas-flow. A bag made of a semipermeable membrane is also very fragile and easily damaged during handling.

EP 0258795 describes a system for cultivating cells comprising an inner bag made of a semipermeable membrane for culturing the cells, housed in an outer bag (container) for the culturing medium and gas. After filling, the system is put on a rotator.

In such a bag-in-a-bag construction, exchange of gases is limited due to the walls they must pass and the length of the diffusion path between the wall and the membrane. This is a severe limitation, if oxygen is a limiting factor for the culturing of the cells. To prevent a shortage of oxygen for the cells, in this system, sterilized air is introduced into one or both bags by the operator via a sterilizing air-filter. Air-bubbles however, are harmful to the cells as they induce shear-stress during shaking. Shear-stress may lead to damage to the membrane and thus to the functioning of the cells. Oxygen is preferably delivered to the cells, dissolved in the medium.

It is, therefore, an object of the present invention to provide an improved bioreactor for culturing of cells for therapeutic use, overcoming the drawbacks of the previously described systems.

Another object of the invention is to provide an improved bioreactor which avoids shear stress, maximizes oxygen delivery to the cells and nevertheless provides the cell culture in an easily harvestable volume.

The present invention comprises a flexible bioreactor container for the culture of cells consisting of two compartments being separated by a semipermeable membrane, thus forming a smaller compartment for cells and a larger compartment for medium, the cell compartment being defined by the semipermeable membrane and a first outer wall of the container, the medium compartment being defined by the semipermeable membrane and a second outer wall of the container, both compartments having at least one port for the introduction or removal of fluids or cell suspensions. The outer wall of the cell compartment is made of a polymer with a high gas permeability.

Cells are stored in the first, small compartment, while medium is stored in the second, large compartment.

This flexible container is essentially manufactured by sealing together three sheets of material:
A first sheet (the outer wall of the cell compartment), made from a highly gas permeable material,
A second sheet, being the semipermeable membrane,
A third sheet (the outer wall of the medium compartment), made from a material having the desired properties for the specific application of the bioreactor.

Generally the compartment for the growth medium will be much larger than the cell compartment to serve as a large source for the nutrients and supply a large volume for the dilution of toxic waste products from the cell compartment. The volume in the cell compartment will be less than 10% of the volume of the medium compartment.

After introduction of the medium in the large compartment, the cells are introduced in the small compartment.

The cells are contained in a relatively small compartment in the bioreactor covering a very large surface area. On one side, the compartment is defined by the gas-permeable sheet, thus allowing for direct gas exchange for the cells over a short diffusion path. On the other side, this compartment is defined by the semipermeable membrane. The fluid that is contained in the medium compartment delivers nutrients to the cells and removes waste products from the cells over this membrane. Although the main source of oxygen is the outer wall of the cell compartment, also on the outer wall of the medium compartment some gas exchange will take place. Thus, the medium will also supply some oxygen to the cells.

The invention brings the cells in a thin layer in a chamber that combines:
a good exchange of nutrients and waste products by means of a large volume of medium from one wall (the semipermeable membrane) and
a good exchange of gases over the other wall, which is the high gas permeable outer wall of the cell compartment.

Both compartments are fitted with at least one port for the introduction of material. This can be a standard injection port or a hermetically-sealed tube such as used for sterile docking.

The cell suspension and the medium are introduced for culturing in this bioreactor in their respective compartments. Both compartments are kept essentially free from air bubbles. Subsequently, the container is stored for culturing. Culturing usually takes place in an incubator under controlled conditions for temperature (usually between 32 and 37°C) and atmosphere (usually 95% O₂ with 5% CO₂). The bag can be gently shaken, turned or rotated for homogenization of the fluids, thus allowing for optimum exchange of nutrients, gases and waste products without inducing shear stress on the cells.

Shakers that are commonly used for the storage of platelets in blood banks may be used for this purpose. As the volume of medium is far in excess compared to the volume in the cell compartment, there is a large excess of nutrients available for the cells. At the same time, there is a large volume available for the dissolution of the waste products. This leads to relatively low concentrations of the waste products in the medium and a build-up of toxic concentrations of waste products is slowed down. A constant inflow of medium in the medium compartment (requiring pumps and operator attention etc.) is therefore not needed. The medium can be changed if necessary by draining the medium compartment and introducing new medium without affecting the cell compartment. During incubation, the ports of the bioreactor are preferably hermetically sealed, thus preventing microbial contamination.

After incubation for the required time, the cells can be easily harvested in a small volume from the cell compartment by direct withdrawal. Subsequently they are purified and may be injected into the patient.

The invention also comprises a method in which the container is used for the culture of the cells.

According to the invention, the flexible container for the cells consists of two compartments separated by the semipermeable membrane and defined between the semipermeable membrane and the gas-permeable outer wall, one of these compartments having a relatively small volume and the other a relatively large volume, the semipermeable membrane being a wall common to both of these compartments.

According to the method aspect of the invention, the container with the cell culture in the small compartment and the nutrient medium in the large compartment is incubated in a vessel in which the container is subjected to slight movement which does not generate detrimental shear in an oxygen-containing environment in this vessel, the gas exchange within the vessel carrying off carbon dioxide which diffuses through the outer walls of the flexible container, the incubation vessel being maintained at an incubation temperature of 32 to 37°C.

The above and other objects, features, and advantages will become more readily apparent from the following description, reference being made to the accompanying drawing in which:
FIG. 1 is an elevational view which shows a bioreactor according to our invention;
FIG. 1A is a cross section of this bioreactor at the port of the cell compartment, taken along line IA-IA of FIG. 1;
FIG. 1B is a cross section of this bioreactor at the port of the medium compartment taken along line IB-IB of FIG. 1;
FIG. 2 is a perspective view showing the three layers of material which can be used in the fabrication of the bioreactor of FIGS. 1, 1A and 1B;
FIG. 3 is an elevational view which shows a bioreactor container with the outer wall of the cell compartment sealed to the semipermeable membrane in a special pattern;
FIG. 4 is an elevational view of an embodiment of similar construction but formed with another pattern;
FIG. 5 is a cross section of the container of FIG. 4, taken along line IVA-IVA of FIG. 4;
FIG. 6 is a perspective view which shows a semipermeable membrane supported by supporting layers;
FIG. 7 is an elevational view, partly broken away, which shows a bioreactor container fitted with hermetically sealed tubing for the introduction or removal of fluid or cell suspension in a sterile manner by means of a sterile connection device;
FIG. 7A is a section along line VIIA-VIIA of FIG. 7;
FIG. 7B is a section along line VIIB-VIIB of FIG. 7; and
FIG. 8 is a diagram of an incubation vessel receiving the container of the invention and illustrating the application of the method of the invention.

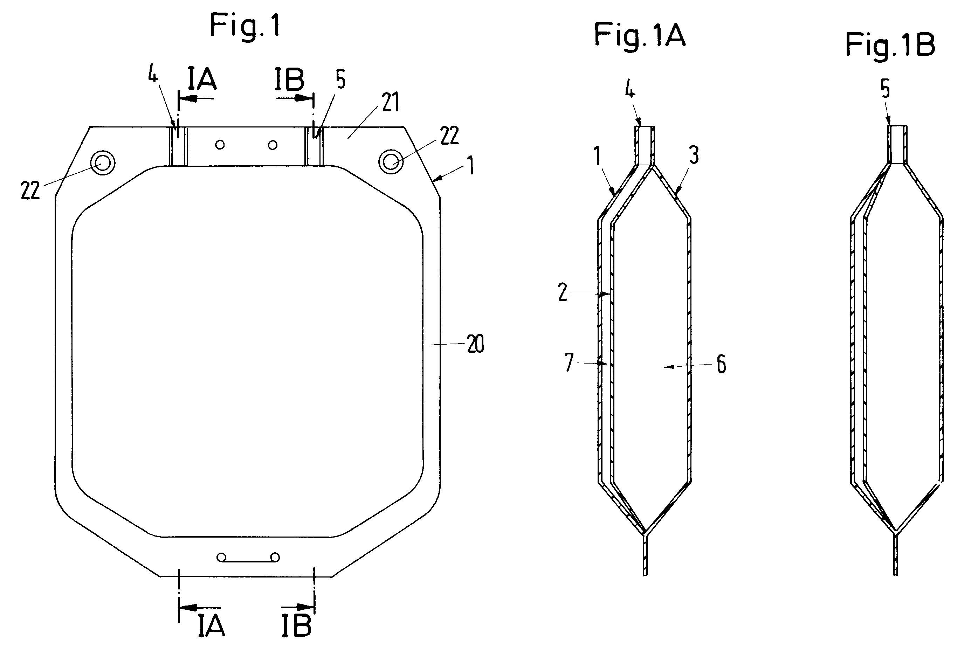

FIG. 1 shows a bioreactor according to our invention existing of two compartments 6, 7, separated by a semipermeable membrane 2.

FIG. 1A shows a cross section of this bioreactor at the place of port 4.

FIG. 1B shows a cross section of this bioreactor at the place of port 5.

The outer wall of the bioreactor 1 defining the cell compartment 7 of the present invention is made of a flexible material with a high gas permeability. For proper culture conditions of the cells it is essential that this sheet has a good permeability for oxygen and preferably also for CO₂. This sheet forms the main membrane for the exchange of oxygen to the cells. Materials that may meet these specifications are TOTM plasticized PVC foils, polyether-esters, fluorine-containing polymers such a polytetrafluoroethylene, silicones, poly(ethylene-vinyl acetate), polyolefins and modified polyolefins such as polypropylene-elastomer compounds. Additionally, the total gas permeability of the cell compartment is influenced by the thickness of the film, a possible surface structure and the total surface area of the cell compartment to be covered. For the ease of handling of the bioreactor, it is essential that the material is flexible, thus preventing a build-up of pressure due to release of CO₂ during culturing. The sheet can either be a monolayer or a multilayer film or an anisotropic film.

The semipermeable membrane 2 is a membrane such as used for dialyses. It can be manufactured from well-known materials such as cellulose or its esters, polysulfone, polycarbonate etc. This cut-off is 5 to 20kD (kilodalton), preferably around 10kD. Cells will thus stay in the cell compartment. Also, larger molecules such as proteins and hormones cannot pass this membrane. They are therefore added to the cell compartment. As the cell compartment has a small volume in comparison to the medium compartment, considerable saving can be achieved on these generally expensive components. Smaller molecules such as the nutrients will pass this membrane.

The outer wall of the bioreactor 3 defining the medium compartment 6 of the present invention is made of a flexible material. Generally, this sheet will have an appreciable gas permeability to supply the culture medium with oxygen. As the main membrane for oxygenation of the cells is however formed by the sheet on the side of the cell compartment, this sheet is used to optimize the total performance of the bioreactor such as ease of handling, sturdiness, temperature resistance etc. Thus, depending upon the requirements of the bioreactor, a sheet material differing in chemical or physical characteristics from the outer wall of the cell compartment may be employed for this wall. This material is flexible, to prevent a build-up of pressure during culturing and this sheet may either be a monolayer or a multilayer sheet. Plastics that can be used include PVC, plasticized with plasticizer such as DEHP or TOTM or citrate, polyether-esters, fluorine-containing polymers such as polytetrafluoroethylene, silicones, poly(ethylene-vinyl acetate), polyolefins and modified polyolefins such as polypropylene-elastomer compounds.

From FIGS. 1, 1A and 1B, it can be seen that the flexible bag forming the bioreactor 1 can be formed from a number of sheets which can include the partition 2 between the compartments 6 and 7 and which is a semipermeable membrane, and the outer wall sheets 3 which are permeable to oxygen and carbon dioxide, fused together along the margin of the bioreactor to form a seam 20 which, along the top portion 21, can be provided with holes 22 for suspending the bag from a suitable support. In the region of the ports 4 and 5, the fused seam 20 is interrupted. In that sense, the bioreactor has a configuration of a blood bag or a bag for administrating fluids to a patient.

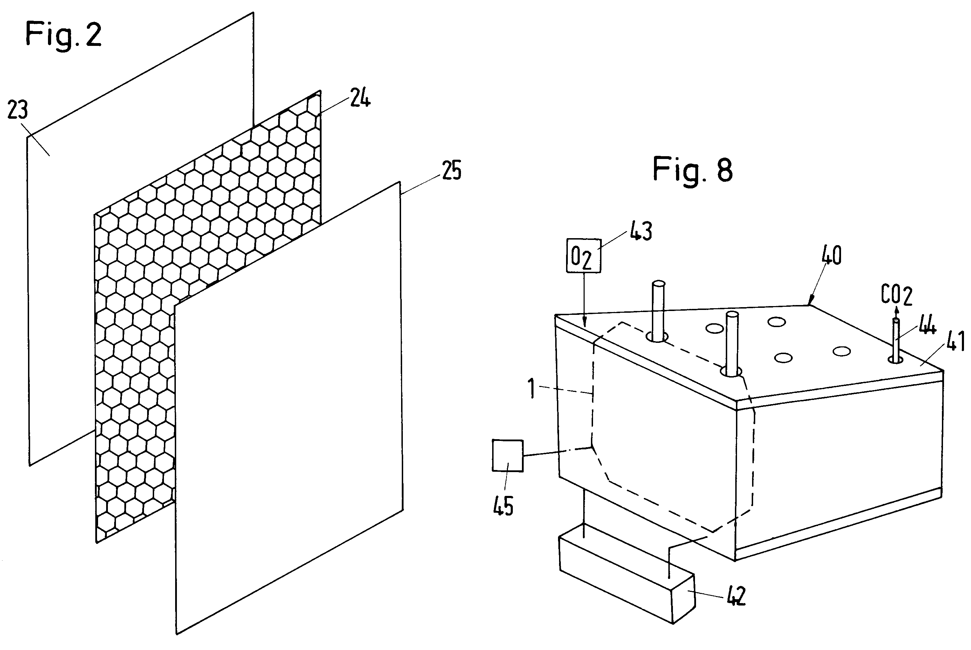

FIG. 2 shows the three layers of material that form the basic elements of the container.

More particularly, FIG. 2 shows a sheet 23 forming one outer wall of the bag 1 of FIGS. 1, 1A and 1B, namely, the outer wall of the cell compartment 7, the semipermeable membrane 24 adapted to form the partition 2 between the cell compartment and the medium compartment 6, and the sheet 25 adapted to form the outer wall of the medium compartment 6, these sheets and the membrane being thermally or ultrasonically fusible together to form the seam 20. The semipermeable membrane 24 has been shown to be inscribed in a hexagonal pattern in FIG. 2 to distinguish it from the outer wall sheets which, as has been stated, are permeable to oxygen and carbon dioxide, but not to the liquid medium or the cell culture.

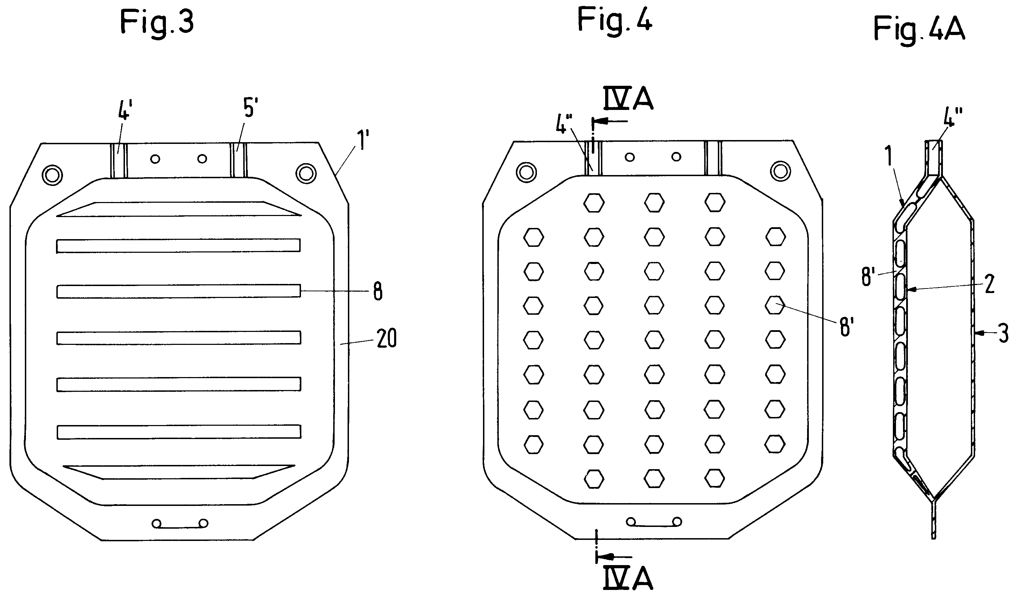

FIGS. 3 and FIGS. 4 and 4A show two other embodiments of this invention.

Generally it is desirable to culture cells in a well-defined compartment in which the diffusion paths for the nutrients and the gases between the cells and the wall and the inner membrane are very identical within the compartment. The more uniform the distribution of these nutrients and waste products in the cell compartment is, the more uniform are the culture conditions and the easier it is to validate the culture process. This prevents heterogeneous cell populations. In this invention this is achieved by bringing the cells together in a relatively thin layer between the semipermeable membrane that separates the cells from a large volume of medium and a large membrane that offers good oxygenation to the cells.

To form a uniform and thin layer the outer wall of the cell compartment and the membrane may be sealed partly together to define more precisely the maximum distance between the wall and the semipermeable membrane and thereby the diffusion distances for the gases, nutrients and waste products. Such a pattern can be achieved by direct sealing of the semipermeable membrane in the desired pattern to the wall of the container. Also, the outer wall of the cell compartment can be manufactured with a surface structure to which the semipermeable membrane is bound.

From FIG. 3 it will be apparent that the bioreactor 1', which can otherwise have the configuration shown in FIGS. 1, 1A and 1B, with the ports 4' and 5' and the peripheral seal 20, can have its outer wall of the cell compartment 5 heat-sealed or ultrasonically sealed to the membrane 2 along ribs 8 which subdivide the cell compartment into horizontal zones as shown generally in FIG. 4A. The ribs 8 may be surface structures formed in the outer wall of the cell compartment as has been mentioned previously.

In FIGS. 4 and 4A, the outer wall of the cell compartment is sealed at locations 8' to the membrane 2, the locations 8' being shown to have a hexagonal configuration in the embodiment of FIGS. 4 and 4A and to be spaced apart both vertically and horizontally. Other patterns of fusion of the outer wall of the cell compartment to the semipermeable membrane may be used as well and the number of sealing locations can be selected to reduce the available volume of the cell compartment to that which is desired.

Regardless of the patterns used, however, the spaces defined between the outer wall of the cell compartment and the semipermeable membrane should all communicate with one another to afford access through the port 4''.

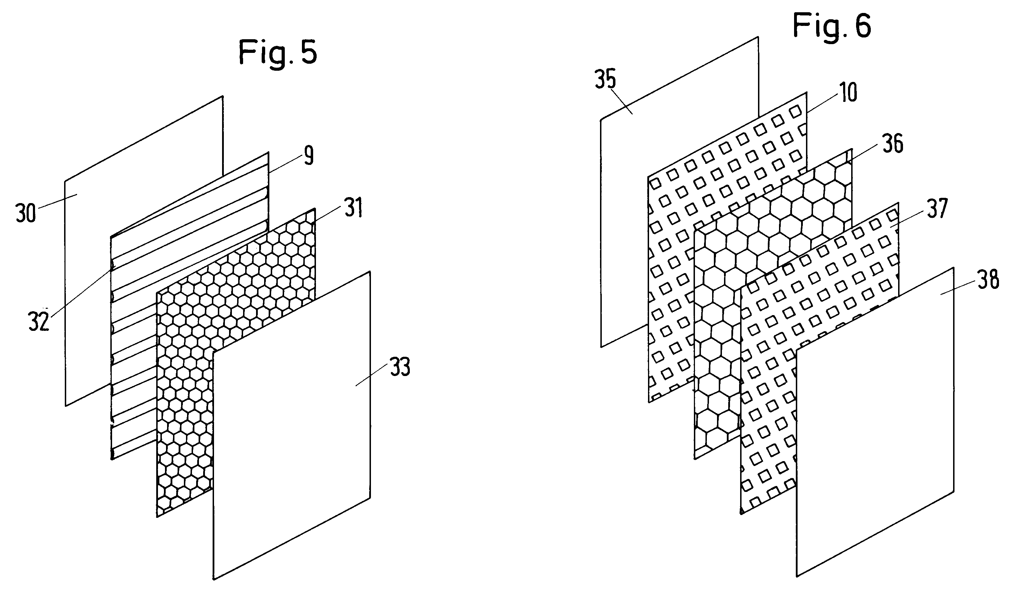

FIG. 5 shows, alternatively, a special layer 9 that contains the desired pattern and that can be sealed between the walls 1, 2 of the cell compartment.

In FIG. 5, we show a stack of layers for forming the bioreactor in accordance with another embodiment of the invention. Here, between the sheet 30 forming the outer wall of the cell compartment and the semipermeable membrane 31 forming another wall of the cell compartment and the partition between the cell compartment and the nutrient medium compartment, the pattern layer 9 is provided which has ribs 32 which can be fused, for example, to the sheets 30 and 31 to break up the cell compartment. Of course, the ribs will not be fused to the sheets 30 and 31 over their entire lengths, thereby ensuring communication between the pockets of the cell compartment which are thus formed. The outer wall of the medium compartment is shown at 33 in FIG. 5.

FIG. 6 shows a semipermeable membrane 2 supported by a protection net 10 to prevent damage to the precious membrane due to pressure differences between the compartments or rough handling This protective support 10 may either be placed in the cell compartment 7 or in the medium compartment 6 or in both compartments.

Also, a pattern can be introduced into the medium compartment to define a flow pattern for the medium and thus aid the even distribution of nutrients and waste products in the medium compartment.

In FIG. 6, more specifically, the net 10 disposed between the outer wall sheet 35 and the semipermeable membrane 36 can be fused to the semipermeable membrane to provide a support for it. A further net or mesh support layer can be provided at 37 between the semipermeable membrane 36 and the sheet 38 forming the outer wall of the medium compartment. The nets 10 and 37 thus not only provide support for the semipermeable membrane and limit any danger of rupture in handling, but they also serve to distribute the cell culture along the semipermeable membrane and to distribute the nutrient medium along the semipermeable membrane.

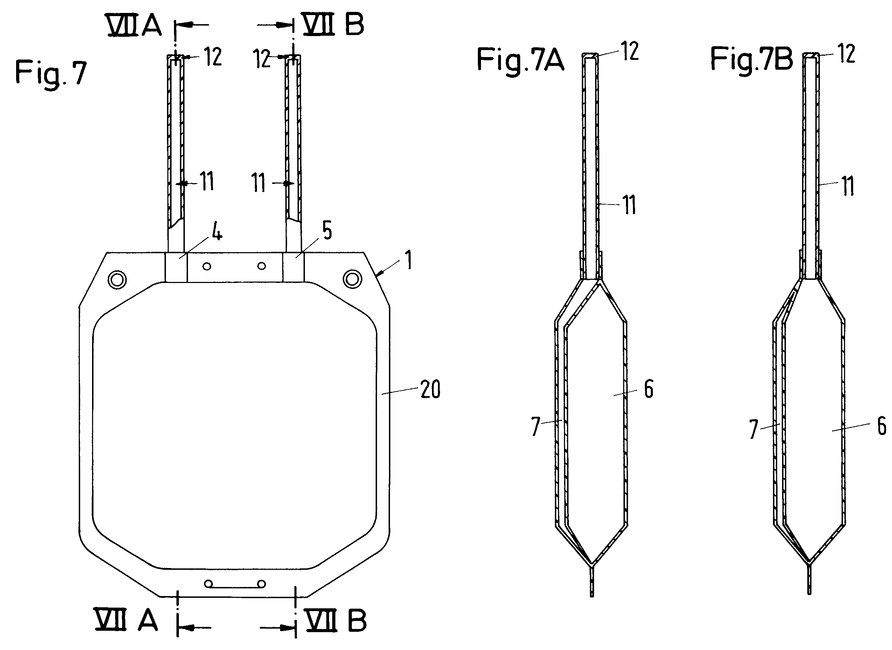

FIG. 7 shows the container with at least one port, fitted with a piece of tubing 12 that is hermetically sealed at 11 for the introduction or removal of fluid or cell suspension in a sterile manner by means of a sterile connection device.

Finally, both compartments can be fitted with more than one port. For instance, the cell compartment may be fitted with two ports, one for the introduction of the cell suspension and one for the harvesting/sampling. Also the medium compartment can be fitted with two ports: one for the introduction and one for the withdrawal of medium. The ports may be adjacent or they may be placed at the opposite sides of the compartments.

From the above description it will be clear that the bioreactor of the present invention offers many advantages over existing bioreactors.

One essential advantage being, that the cells are kept in a separate compartment directly in contact with a large medium compartment and directly under the outer wall of the container, thus allowing an optimum exchange of O₂/CO₂ and nutrients with short diffusion paths. Introduction of air bubbles in the medium- or cell compartment is not required. In this way, high cell densities can be reached. Therefore, the number of containers required to culture the minimum number of cells needed for patient treatment is reduced, and thus the number of aseptic handlings.

The flexible container prevents the build-up of pressure. By providing the short diffusion paths, heterogeneity of the medium in the cell-population is prevented. In this bioreactor, expensive hormones and proteins can be injected directly into the cell compartment without leakage to the medium compartment, thus producing considerable cost savings. The semipermeable membrane also protects the cells against a possible contamination from the medium.

The bioreactor does not require a constant in- and outflow of medium during incubation of the cells. And as it is completely closed during operation, relatively little operator attention is required. The flexible bioreactor allows for easy handling, without the build-up of pressure and the need to release pressure in between medium changes.

Because the cells are contained in a relatively small volume in the cell compartment, harvesting is easy and does not require extensive concentration procedures.

In FIGS. 7, 7A and 7B, more specifically, it will be seen that the tubes 11 are fused within the ports 4 and 5 of the bioreactor 1 which is provided with the seam 20, the cell compartment 7 and the medium compartment 6 as previously described in connection with FIGS. 1 and 1A.

In a sterile docking procedure, the seal 12 of tubes 11 may be sterilely removed and the tubes 11 fused to tubes for supplying medium to or withdrawing medium from the bioreactor or for introducing the cell culture or dispensing it, respectively.

As can be seen from FIG. 8, the bioreactor 1 can be suspended in a vessel 40 which can have a removable cover 41 and a temperature control system 42 for thermostating the vessel at the incubation temperature of 32 to 37°C.

Oxygen is supplied to the space around the bioreactors 1 via an oxygen supply system 43 and carbon dioxide is discharged in the residual oxygen flow as represented at 44. A mild shaking action can be imparted to the bag via the bag shaker 45 shown diagrammatically in FIG. 8.

With this system, therefore, oxygen diffuses through the outer walls into both the medium and the cell culture and thus into the cell culture also through the semipermeable membrane from the medium while carbon dioxide can diffuse in both directions through the outer wall of the culture compartment and into the medium through the semipermeable membrane, when carbon dioxide can diffuse from the medium into the space around the bioreactor to be swept from the incubator.

When the culture is complete, it can be dispensed directly to the patient or otherwise forced out of the bioreactor through the port 4, 4' and 4'' and in tube 11 connected therewith.

## Claims

1. A flexible container for the culture of cells consisting of two compartments being separated by a semipermeable membrane, thus forming a relatively small compartment for cells and a relatively large compartment for medium, the cell compartment being defined by said semipermeable membrane and a first outer wall of said container, the medium compartment being defined by said semipermeable membrane and a second outer wall of said container, both compartments having at least one port for the introduction or removal of fluids or cell suspension.

2. The container defined in claim 1 in which the cut-off of the semipermeable membrane is between 5 and 20 kD.

3. The container defined in claim 2 in which the outer wall of said cell compartment is made of a flexible plastic with a high gas permeability selected from TOTM plasticized PVC, polyether-ester, fluorine-containing polymers, silicone, poly(ethylene-vinyl acetate), polyolefin and a modified polyolefin compound with a high gas permeability in the form of a single- or a multi-layer sheet or in the form of an anisotropic film.

4. The container defined in claim 3 in which said outer wall of said medium compartment is made of a flexible plastic selected from DEHP or TOTM or citrate plasticized PVC, polyether-ester, fluorine-containing polymers, silicone, poly(ethylene-vinyl acetate), polyolefin and a modified polyolefin compound in the form of a single- or a multi-layer sheet.

5. The container defined in claim 4 in which at least one port is fitted with a hermetically sealed tube for the introduction or removal of fluid or cell suspension in a sterile manner by means of a sterile connection device.

6. The container defined in claim 4 in which said semi-permeable membrane that separates the compartments is partially bound to said wall of said cell compartment.

7. The container defined in claim 4 in which said cell compartment is fitted with a spacer to which said outer wall and said membrane are bound.

8. The container defined in claim 4 in which said compartment is fitted with a spacer to which said outer wall and said membrane are bound.

9. The container defined in claim 4 in which said semipermeable membrane is protected with a supporting net on one or both of the sides of said cell- or medium compartments.

10. The container defined in claim 4 in which one or both compartments contain two or more ports.

11. The container defined in claim 2 wherein the cut-off of the semipermeable membrane is about 10 kD.

12. A bioreactor comprising a flexible container formed with a cell compartment of small volume defined between a gas-permeable outer wall and a semipermeable membrane with a cut-off of about 10 kD, and a culture medium compartment of relatively large volume defined between said semipermeable membrane and a gas-permeable outer wall, said walls and said membrane being fused together around a periphery of said container to form a seam, respective ports extending through said seam and communicating with said compartment.

13. The bioreactor defined in claim 12, further comprising respective tubes sealed in said ports and provided with respective seals hermetically closing said tubes.

14. A method of culturing cells which comprises the steps of:
(a) providing a bioreactor in the form of a flat flexible container;
(b) a bioreactor comprising a flexible container formed with a cell compartment of small volume defined between a gas-permeable outer wall and a semipermeable membrane with a cut-off of about 10 kD, and a culture medium compartment of relatively large volume defined between said semipermeable membrane and a gas-permeable outer wall, said walls and said membrane being fused together around a periphery of said container to form a seam, respective ports extending through said seam and communicating with said compartment, introducing a cell mass to be cultured in said cell compartment;
(c) introducing a culture medium into said medium compartment; and
(d) introducing said bioreactor into a vessel thermostated to an incubation temperature of 32 to 37°C feeding oxygen into said vessel and removing carbon dioxide therefrom whereby oxygen permeates into said bioreactor through said walls and carbon dioxide diffuses from said bioreactor through said wall, thereby providing cell growth in said cell compartment.

15. The method defined in claim 14, further comprising the step of moving said bioreactor in said vessel.
